(19) Europäisches Patentamt / European Patent Office / Office européen des brevets

(11) **EP 2 319 415 A1**

(12) **EUROPEAN PATENT APPLICATION**
published in accordance with Art. 153(4) EPC

(43) Date of publication:
**11.05.2011 Bulletin 2011/19**

(51) Int Cl.:
*A61B 8/00* (2006.01)    *G01N 29/00* (2006.01)

(21) Application number: **09809941.9**

(22) Date of filing: **26.08.2009**

(86) International application number:
**PCT/JP2009/064857**

(87) International publication number:
**WO 2010/024290 (04.03.2010 Gazette 2010/09)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **27.08.2008 JP 2008217746**

(71) Applicant: **Canon Kabushiki Kaisha Tokyo 146-8501 (JP)**

(72) Inventors:
• **FUKUTANI, Kazhiko (JP)**
• **NAKAJIMA, Takao (JP)**
• **BABA, Yoshitaka (JP)**

(74) Representative: **TBK-Patent Bavariaring 4-6 80336 München (DE)**

(54) **DEVICE FOR PROCESSING PHOTO ACOUSTIC INFORMATION RELATING TO LIVING BODY AND METHOD FOR PROCESSING PHOTO ACOUSTIC INFORMATION RELATING TO LIVING BODY**

(57) A biological information processing apparatus includes: a light source 11 that irradiates light 12 to a light irradiation region 13A on a test object 13; an acoustic wave detector 17 that detects an acoustic wave 16 generated by a light absorber 15 in the test object upon its absorption of the light, and outputs a detection signal; and an electronic control system 18 having an amplifier that amplifies the detection signal outputted from the acoustic wave detector 17. The electronic control system controls a gain of the amplifier in such a manner that a gain for a detection signal of an acoustic wave generated at a first location in the test object becomes larger as compared with a gain for a detection signal of an acoustic wave generated at a second location which exists nearer to the light irradiation region than the first location does.

FIG. 1

**Description**

Technical Field

The present invention relates to a biological information processing apparatus and a biological information processing method.

Background Art

In general, many imaging apparatuses using X-rays, ultrasonic waves, and MRI (nuclear magnetic resonance imaging) are used in the medical field. On the other hand, in the medical field, studies are being positively carried out on an optical imaging apparatus which obtains information in a living body by causing a beam of light irradiated from a light source such as a laser to propagate in a test object such as a living body, and detecting the propagation light or the like. As one of such optical imaging techniques, there has been proposed photoacoustic tomography (PAT) (Non Patent Literature 1).
The PAT is a technique in which a test object is irradiated with pulsed light generated from a light source, and acoustic waves generated from a living body tissue, which has absorbed the energy of the light propagated and diffused in the interior of the test object, are detected in a plurality of locations, so that those acoustic waves or signals are subjected to analysis processing to visualize the information related to optical property values in the interior of the test object. According to this, an optical property value distribution, especially a light energy absorption density distribution, in the test object can be obtained.
According to the Non Patent Literature 1, in photoacoustic tomography, an initial sound pressure ($P_0$) of a photoacoustic wave generated from an absorber in the test object by light absorption can be represented by the following formula.

$$P_0 = \Gamma \cdot \mu_a \cdot \Phi \quad \text{formula (1)}$$

Here, $\Gamma$ is a Grüneisen coefficient and is the product of a coefficient of thermal expansion ($\beta$) and the square of an acoustic velocity (c) divided by a specific heat at constant pressure ($C_P$). Here, $\mu_a$ is the light absorption coefficient of the absorber, and $\Phi$ is the amount of light (this being the amount of light irradiated on the absorber and being also called optical fluence) in a local region. Because it is known that $\Gamma$ will take an almost constant value if the tissue is decided, the product of $\mu_a$ and $\Phi$, i.e., a light energy absorption density distribution, can be obtained by measuring and analyzing the change of the sound pressure P, which is the magnitude of an acoustic wave, in a plurality of locations.

Citation List

Non Patent Literature

Non Patent Literature 1: M. Xu, L. V. Wang, "Photoacoustic imaging in biomedicine", Review of scientific instruments, 77, 041101(2006)

Summary of Invention

Technical Problem

In the photoacoustic tomography, as can be seen from the above-mentioned formula (1), in order to calculate the distribution of the absorption coefficient ($\mu_a$) in the test object, it is necessary to calculate the distribution of the amount of light ($\Phi$) irradiated on the absorber, which generates photoacoustic waves, from the result of the measurement of the sound pressure (P) by means of a certain method. However, because the light introduced in the test object (inparticular, the livingbody) is diffused strongly, an estimation of the amount of light irradiated on the absorber is difficult. Therefore, in the past, it is possible to image, based on the measurement result of the sound pressure of the acoustic wave, only the distribution of light energy absorption density ($\mu_a \times \Phi$) or the distribution of the initial sound pressure ($P_0$) which is obtained by multiplying the light energy absorption density by $\Gamma$. In other words, there has been a problem that light absorbers of the same size, shape and absorption coefficient will be expressed with different contrasts due to the influence of a fluence distribution in the living body (i.e., depending upon where the light absorbers exist in the living body).
In view of the aforementioned problem, the present invention has for its object is to provide a technique for

2

obtaining an image on which the influence of a fluence distribution in the test object is reduced in photoacoustic tomography. In addition, a further object of the present invention is to provide a technique for imaging light absorbers of the same size, shape and absorption coefficient with almost the same contrast, not depending on the existing positions thereof in photoacoustic tomography.

Solution to Problem

In order to achieve the above-mentioned object, the present invention adopts the following construction.

A biological information processing apparatus according to the present invention includes: a light source that irradiates light to a light irradiation region on a test object; an acoustic wave detector that detects an acoustic wave generated by a light absorber in the test object upon its absorption of the light, and outputs a detection signal; an amplifier that amplifies the detection signal outputted from the acoustic wave detector; a control part that controls a gain of the amplifier; and a signal processing part that obtains information on an interior of the test object based on the signal amplified by the amplifier, wherein the control part controls the gain of the amplifier in such a manner that a gain for a detection signal of an acoustic wave generated at a first location in the test object becomes larger as compared with a gain for a detection signal of an acoustic wave generated at a second location which exists nearer to the light irradiation region than the first location does.

A biological information processing method according to the present invention includes: a step of detecting an acoustic wave generated by a light absorber in a test object upon its absorption of light irradiated to a light irradiation region on the test object, and outputting a detection signal; a step of amplifying the outputted detection signal by means of an amplifier; and a step of obtaining information on an interior of the test object based on the signal amplified by the amplifier, wherein in the step of amplifying the detection signal by means of the amplifier, the gain of the amplifier is controlled in such a manner that a gain for a detection signal of an acoustic wave generated at a first location in the test object becomes larger as compared with a gain for a detection signal of an acoustic wave generated at a second location which exists nearer to the light irradiation region than the first location does.

Advantageous Effects of Invention

According to the present invention, in photoacoustic tomography, it is possible to obtain an image on which the influence of a fluence distribution in a test object is reduced.

Brief Description of Drawings

[Fig. 1] Fig. 1 is a view showing a biological information processing apparatus according to a first embodiment of the present invention.

[Fig. 2] Fig. 2A is a view showing conventional biological information processing apparatus, Fig. 2B shows an example of an acoustic wave signal obtained by a conventional apparatus, Fig. 2C shows an example of an optical property value distribution image obtained in a conventional apparatus, and Fig. 2D shows an example of the absorption coefficient distribution of a test object.

[Fig. 3] Fig. 3A is a view showing the biological information processing apparatus according to the first embodiment of the present invention, Fig. 3B shows an example of the gain of an amplifier in the first embodiment, Fig. 3C shows an example of an acoustic wave signal after amplification, and Fig. 3D shows an example of an optical property value distribution image reconstructed from the signal of Fig. 3C.

[Fig. 4] Fig. 4 is a view showing an electronic control system in the first embodiment of the present invention.

[Fig. 5] Fig. 5A is a view showing a biological information processing apparatus according to a second embodiment of the present invention, and Fig. 5B shows an example of the gain of an amplifier in the second embodiment.

[Fig. 6] Fig. 6 is a flow chart showing signal processing carried out by the biological information processing apparatus according to the second embodiment of the present invention.

[Fig. 7] Fig. 7 is a view showing a biological information processing apparatus according to a third embodiment of the present invention.

[Fig. 8] Fig. 8A is a fluence distribution on an axis a of Fig. 7, Fig. 8B is a fluence distribution on an axis b of Fig. 7, Fig. 8C is a gain which is provided to a detection element on the axis a, and Fig. 8D shows a gain which is provided to a detection element on the axis b.

[Fig. 9] Fig. 9 is a view showing an electronic control system in the third embodiment of the present invention.

Description of Embodiments

A biological information processing apparatus according to this embodiment is an imaging apparatus using photoacoustic tomography (PAT). This biological information processing apparatus is provided with a light source that irradiates light onto a light irradiation region on a test object, and an acoustic wave detector that detects acoustic waves (including ultrasonic waves) generated at the time when a light absorber in the test object absorbs the light, and outputs a detection signal. In addition, the biological information processing apparatus is provided with an amplifier that amplifies the detection signal outputted from the acoustic wave detector, a control part that controls the gain of this amplifier, and a signal processing device that images information (optical property value distribution) on an interior of the test object based on the signal amplified by the amplifier. Because the light having entered the interior of the test object from the light irradiation region is diffused within the test object, the amount of light (the number of photons) decreases remarkably in accordance with an increasing distance from the light irradiation region. That is, when a comparison is made between a first location in the test object and a second location therein which exists nearer to the light irradiation region than the first location, the amount of light reaching the first location becomes smaller than the amount of light reaching the second location. Even if light absorbers of the same size, shape and absorption coefficient exist at the first location and at the second location, respectively, a difference occurs between the sound pressures of acoustic waves generated at the respective locations under the influence of a fluence distribution (difference in the amount of light) in such a test object. Accordingly, in this embodiment, the control part controls the gain of the amplifier in such a manner that a gain for a detection signal of an acoustic wave generated at the first location becomes larger as compared with a gain for a detection signal of an acoustic wave generated at the second location. According to such gain control, it becomes possible to reduce the influence of the fluence distribution in the test object.

Here, it is desirable that the control part control the gain of the amplifier so as to correct a difference between the amount of light reaching the first location and the amount of light reaching the second location. To correct a difference between the amounts of light means that a difference between the amounts of light after correction becomes smaller than a difference between the amounts of light before correction. It is desirable that the difference between the amounts of light becomes as small as possible, and it is most desirable that the difference between the amounts of light become zero. Because it is difficult to accurately measure and grasp an actual fluence distribution in the test object, in actuality, the control part may assume a pseudo fluence distribution beforehand, and determine the value of the gain in such a manner that the difference between the amounts of light at the respective locations in the test object will become small as much as possible based on the pseudo fluence distribution. According to such gain control, the influence of the fluence distribution in the test object can be reduced as much as possible, whereby it becomes possible to image the light absorbers of the same size, shape and absorption coefficient with almost the same contrast, not depending on the existing positions thereof.

The acoustic wave detector is desirably arranged in such a manner that the distance between the acoustic wave detector and the first location is mutually different from the distance between the acoustic wave detector and the second location. With such an arrangement, there arises a difference between the detection time of the acoustic wave generated at the first location, and the detection time of the acoustic wave generated at the second location. In other words, it becomes possible to estimate, based on the detection time of the acoustic wave by the acoustic wave detector, the position in the test object at which an acoustic wave has been generated. In this case, the control part needs only to change the gain of the amplifier according to the detection time of the acoustic wave by the acoustic wave detector. For example, in cases where the acoustic wave detector is arranged at the opposite side of the light irradiation region, the acoustic wave generated at the first location is detected earlier than the acoustic wave generated at the second location, so the earlier the detection time, the larger the gain is made. On the contrary, in cases where the acoustic wave detector is arranged at the same side of the light irradiation region, the acoustic wave generated at the second location is detected earlier than the acoustic wave generated at the first location, so the later the detection time, the larger the gain is made. According to such simple gain control, the influence of the fluence distribution in the test object can be reduced at a suitable manner.

As for the pseudo fluence distribution in the test object, for example, in cases where a light irradiation region is sufficiently large with respect to that area of the test object which is to be imaged, a distribution can be assumed in which the amount of light decreases in an exponential function manner depending on the distance (depth) from the light irradiation region. In this case, the control part needs only to control the gain of the amplifier in such a manner that the gain is changed in an exponential function manner with respect to the detection time of the acoustic wave by the acoustic wave detector. In addition, in such a case, the fluence distribution in the test object such as a living body can be characterized with an equivalent attenuation coefficient, so it is desirable that an exponent of the above-mentioned exponential function include an average equivalent attenuation coefficient of the test object. According to this, the pseudo fluence distribution in the test object can be modeled in an easy manner. Here, note that in cases where there are a plurality of light irradiation regions or in cases where the shape of the test object is complicated, the pseudo fluence distribution in the test object is estimated by a method to be described later.

It is desirable that the information on the interior of the test object to be imaged by the signal processing device be an absorption coefficient distribution in the interior of the test object. Here, note that the correction of the strength of the detection signal by means of the gain control as stated above is equivalent to correcting the color and density of an image (optical property distribution image) reconstructed by the signal processing device. According to the image thus corrected, it becomes possible to grasp the absorption coefficient distribution in the test object.

It is desirable that the acoustic wave detector be constructed so as to be able to detect acoustic waves at a plurality of locations. In addition, in cases where the test object is a living body, it is desirable to use light of a wave length in the range of not less than 400 nm to not more than 1,600 nm in consideration of the transmittance thereof. The light absorber may be a living body tissue such as a blood vessel, a tumor or the like, or may be a contrast medium that is introduced in the test object.

Hereinafter, preferred embodiments of this invention will be described in detail by way of example with reference to the attached drawings.

[First Embodiment]

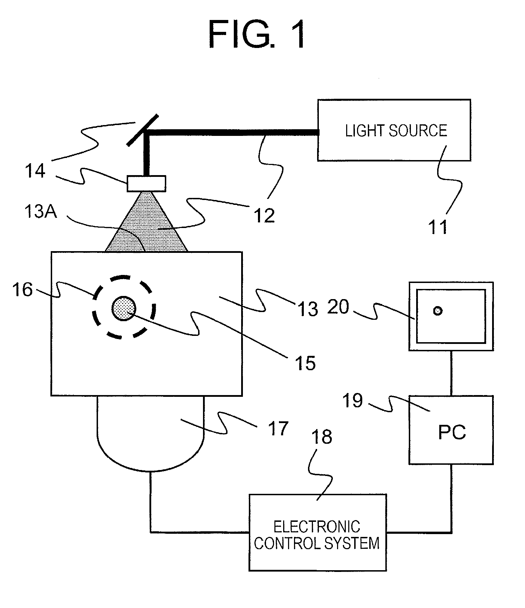

Fig. 1 shows the construction of a biological information imaging apparatus in a first embodiment of the present invention. Now, the first embodiment of the present invention will be described based on Fig. 1. The biological information processing apparatus to be described here is a biological information processing apparatus which makes it possible to image an optical property distribution in a living body and a concentration distribution of substances that constitute a living body tissue obtained from these information, for the purposes of diagnosis of a malignant tumor, a vascular disease, etc., and of the follow-up of a chemical treatment, etc.

The biological information processing apparatus is composed of a light source 11, an optical device 14, an acoustic wave detector (also referred to as a probe) 17, an electronic control system 18, a signal processing device 19, and a display device 20. The light source 11 is a device that emits light 12. The optical device 14 is an optical systemthat is composed of, for example, lenses, mirrors, optical fibers, etc. The light 12 emitted from the light source 11 is guided by the optical device 14, so that it is irradiated on a test object 13 such as a living body. When a part of the energy of the light transmitted through the interior of the test object 13 is absorbed by a light absorber 15 such as a blood vessel, etc., an acoustic wave (ultrasonic wave) 16 is generated from the light absorber 15. The acoustic wave detector 17 is a device that detects the acoustic wave 16 generated from the light absorber 15, and changes an acoustic wave signal thereof into an electrical signal. The electronic control system 18 is a control part that performs amplification, digital conversion, etc., of the electrical signal outputted from the acoustic wave detector 17. The signal processing device 19 acting as a signal processing part is a device that reconstructs an image (biological information image) from a digital signal outputted from the electronic control system 18, and is composed of, for example, a personal computers (PC). The display device 20 is a device that displays a reconstructed image.

In cases where the test object has a flat plate-like shape, as shown in Fig. 1, and a light irradiation region 13A is sufficiently large with respect to a range to be imaged, when the light 12 is irradiated from the light source 11, an initial sound pressure P0 of the acoustic wave 16 generated from the light absorber 15 lying in the living body can be approximately expressed by the following formula.

$$P_0 = \Gamma \cdot \mu_a \cdot \Phi = \Gamma \cdot \mu_a \cdot \Phi_0 \cdot \exp(-\mu_{eff} \cdot r) \quad \text{formula (2)}$$

Here, $\Gamma$ is a Grüneisen coefficient of the light absorber 15; $\mu_a$ is an absorption coefficient of the light absorber 15; $\Phi$ is an amount of local light (an amount of optical flow speed) absorbed by the light absorber 15; $\mu_{eff}$ is an average equivalent attenuation coefficient of the test object 13; and ($\Phi_0$ is an amount of light that has entered the test object 13. In addition, r is a distance from the region (light irradiation region 13A) to which the light from the light source 11 has been irradiated to the light absorber 15, i.e., the depth of the light absorber 15. As can be seen from this formula, the initial sound pressure $P_0$ of the acoustic wave generated by the irradiation of the light is decided from the absorption coefficient $\mu_a$ and the Gruneisen coefficient $\Gamma$, which are inherent property values, and the amount of local light $\Phi$. Moreover, with respect to the amount of local light $\Phi$, it is understood that the amount of light $\Phi_0$ having come into the test object from the light source changes in an exponential function manner with the product of the equivalent attenuation coefficient $\mu_{eff}$ and the distance r as an exponent. Here, in the above-mentioned formula, an assumption is made that the entire amount of light $\Phi_0$ irradiated to the test object from the light source is constant, and light is irradiated to a sufficiently large region with respect to the thickness of the test object, so the light is transmitted through the interior of the test object like a plane wave.

The Grüneisen coefficient ($\Gamma$) is a known value because it is almost constant if the tissue is known. Accordingly, an initial sound pressure generation distribution or the product of the absorption coefficient ($\mu_a$) and the amount of light (Φ) (light energy absorption density distribution) can be calculated by performing the measurement and analysis of the temporal change of the sound pressure (P) detected by the acoustic wave detector 17. Moreover, if the fluence distribution in the test object can be estimated with respect to the light energy absorption density distribution ($\mu_a \cdot \Phi$) finally obtained, it is also possible to obtain an absorption coefficient distribution of the test object. However, it is very difficult to calculate an exact fluence distribution in the test object, so in conventional photoacoustic tomography, a light energy absorption density distribution ($\mu_a \cdot \Phi$) is displayed as an image in many cases.

As explained above, in the conventional photoacoustic tomography, the distribution of the initial sound pressure P0 generated by the irradiation of pulsed light to the test object or the product of the absorption coefficient ($\mu_a$) and the amount of light (Φ) (light energy absorption density distribution) is imaged. In such a display or indication, however, there is a problem that in cases where light absorbers of the same shape, size and absorption coefficient exist in different places in the test object, they are expressed with mutually different brightness levels or colors. This is because the numbers of photons, i.e., the amounts of local light, reaching the individual light absorbers, respectively, are different from one another.

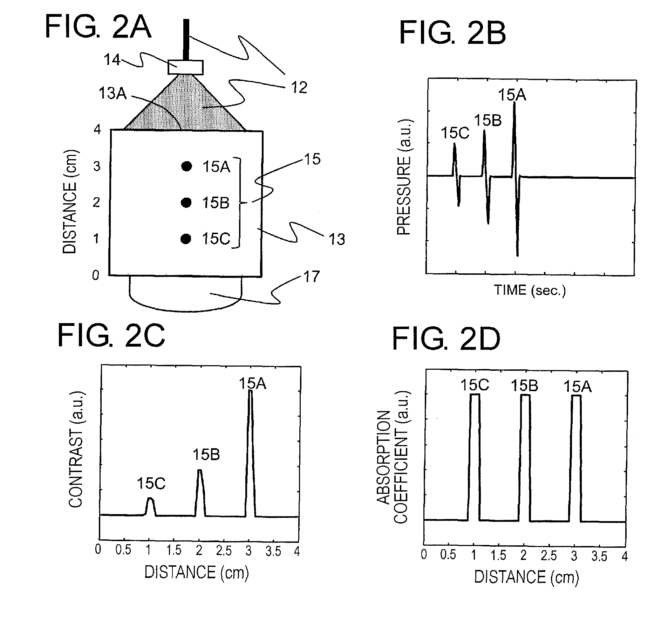

Here, a comparison is made between a conventional photoacoustic tomography apparatus and the photoacoustic tomography apparatus of the present invention. Fig. 2A shows an outline of the conventional photoacoustic tomography apparatus. Here, note that in Fig. 2A, 12 denotes light or pulsed light, 14 denotes an optical device such as lenses, 13 denotes a test object, 15 (15A, 15B, 15C) denotes light absorbers, and 17 denotes an acoustic wave detector. For example, it is assumed that three spherical light absorbers 15A, 15B, 15C exist in the interior of the test object 13, as shown in Fig. 2A. Here, the spherical light absorbers 15A, 15B, 15C are supposed to have a diameter of 2 mm, and the same absorption coefficient. In addition, the light absorbers 15A, 15B, 15C exist at distances of 3 cm, 2 cm, and 1 cm, respectively, from the acoustic wave detector 17, and the pulsed light 12 is irradiated to a place at a distance of 4 cm from the acoustic wave detector 17. That is, the distances (depths) from the light irradiation region 13A to the light absorbers 15A, 15B, 15C are 1 cm, 2 cm, and 3 cm, respectively. In Fig. 2A, the contrasts of the test object 13 and the light absorber 15 indicate differences in the absorption coefficients thereof.

When in the apparatus of Fig. 2A, the test object 13 is irradiated with the pulsed light 12 and a photoacoustic signal is detected by the acoustic wave detector 17, three signals of an N shaped configuration are observed, as shown in an example in Fig. 2B. From Fig. 2B, it is found out that the larger the distance of a light absorber from the light irradiation region, the smaller an acoustic wave corresponding to the light absorber becomes in sound pressure. In addition, it is also found out that the detection time of an acoustic wave is different according to the distance from the acoustic wave detector 17. Here, the number of elements of transducer elements existing in the acoustic wave detector 17 was set to 400, and an element pitch thereof was set to 2 mm.

When ordinary processing is carried out by the electronic control system denoted at 18 of Fig. 1, and an image is formed by the signal processing device 19 of Fig. 1 according to a filtered backprojection algorithm which takes into account the directivity and response of the transducer, an initial sound pressure distribution is imaged. Here, note that the ordinary processing is to amplify each signal with a fixed gain, and to convert it into a digital signal by means of an AD converter. In the signal processing device 19, the above-mentioned image processing is performed based on this digital signal. Fig. 2C is a graph in which the signal strength is represented on an axis on which the light absorbers at that time are arranged in a line. Here, for the sake of a comparison, an actual distribution of the actual absorption coefficient of the test object 13 on the same axis is shown in Fig. 2D. As can be seen from a comparison between Fig. 2C and Fig. 2D, the light absorbers 15A through 15C mutually having the same shape, size and absorption coefficient are expressed at different strengths.

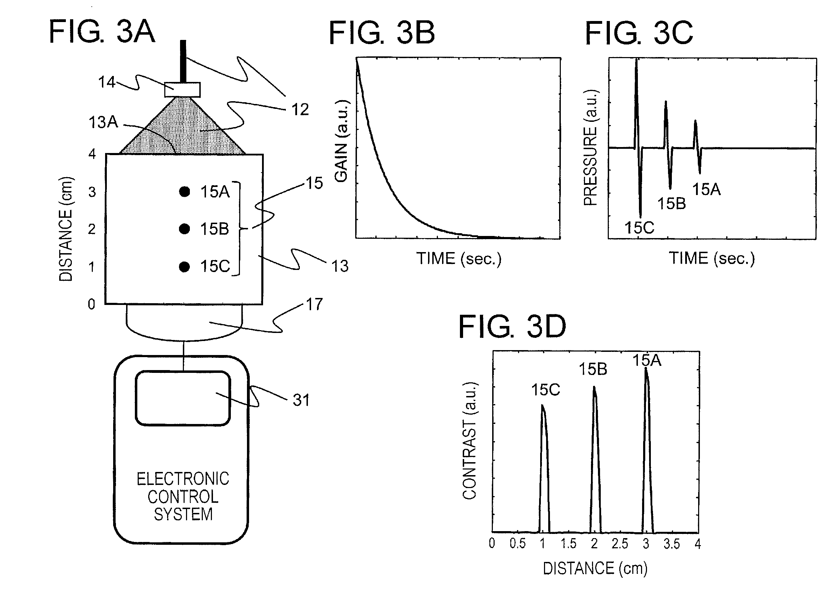

On the other hand, an example of the biological information processing apparatus of this embodiment is shown in Fig. 3A. What in this example is greatly different from the conventional apparatus (Fig. 2A) is that an amplifier 31 of which the gain changes according to the fluence distribution in the test object (difference in the amount of local light) is arranged in the electronic control system at a latter stage or downstream side of the acoustic wave detector 17. Here, note that because the light source and the acoustic wave detector are of the same construction as in the conventional biological information processing apparatus, the signal detected by the acoustic wave detector 17 is the same as that shown in Fig. 2B. However, in this embodiment, by changing the gain of the amplifier 31 according to the detection time of an acoustic wave, the electronic control system (the control part) corrects an influence due to the fluence distribution of the test object.

In the example of Fig. 3A, the acoustic wave detector 17 is arranged at the opposite side of the light irradiation region 13A. Accordingly, the electronic control system estimates the fluence distribution in the test object from formula (2) above, and controls the gain of the amplifier 31 in such a manner that the later the detection time, the smaller the gain becomes in an exponential function manner, as shown in Fig. 3B. This is because the later the detection time of a signal is, i.e., the more distant a light absorber from which the signal comes is from the acoustic wave detector 17, the larger the amount of light to be irradiated becomes, and hence, the higher the sound pressure of an acoustic wave generated by the signal becomes.

Here, an average equivalent attenuation coefficient of the test object is used as an exponent in the exponential function. By doing so, a signal after having been amplified by the amplifier 31 becomes the product of a value shown in Fig. 2B and a corresponding value shown in Fig. 3B, as shown in Fig. 3C, and thus is greatly different from the value shown in Fig. 2B. Here, note that in Fig. 3C, the reason why the amplitude of an acoustic wave at a side near the acoustic wave detector (i.e., an acoustic wave that reaches the detector at an early time) is large is that a photoacoustic wave generated from a light absorber spreads in a spherical wave-like fashion due to a diffraction effect thereof, and the energy per unit area thereof decreases in accordance with the distance of propagation thereof. In addition, it is known that such an attenuation of the acoustic wave is proportional to the distance of propagation thereof, so it is usually corrected at the time of image reconstruction. Finally, the signal obtained in this way is converted into an image by the same image reconstruction processing as in the conventional photoacoustic tomography apparatus. Fig. 3D is a graph in which the strength of light at that time on an axis on which the light absorbers are located in a line. It is understood that an image close to Fig. 2D which is an actual light absorption coefficient distribution can be obtained as compared with Fig. 2C which is a conventional photoacoustic tomography image. Here, it is to be noted that the recomposed image is affected by the influence of the property (directivity, bandwidth, etc.) of the acoustic wave detector and the range in which an acoustic wave can be detected, so it does not completely match Fig. 2C.

In this manner, it becomes possible to obtain an image close to the absorption coefficient distribution instead of the initial sound pressure distribution by performing signal amplification on the time varying signal of the acoustic wave obtained by the acoustic wave detector based on a pseudo fluence distribution of the test object.

Here, note that in the conventional ultrasonic diagnostic apparatus, an amplification factor for a signal of an ultrasonic echo obtained by the acoustic wave detector is controlled to be changed in accordance with the detection time threof. However, the purpose of it is to correct the attenuation of an ultrasonic wave due to its absorption in accordance with the frequency and the distance of propagation thereof, but not to correct a fluence distribution (a difference in the amount of local light) in the test object as in this embodiment. In the photoacoustic wave, a frequency is generated which depends on the magnitude of a light absorber. For example, in the case of assuming a light absorber of about 1 to 2 mm, the frequency to be generated is a low frequency of about 1 MHz, so the amount of attenuation due to the absorption thereof is limited. However, depending on an object to be measured, there may be emitted therefrom an acoustic wave of such a high frequency, the attenuation of which itself can not be disregarded. In such a case, it is also desirable for the electronic control system of this embodiment to perform gain control in consideration of both the correction of the fluence distribution and the correction of the attenuation due to the absorption of the acoustic wave.

Here, note that the fluence distribution in a depth direction in the test object shown at this time is only an example. The relation between the detection time (measuring time) of an acoustic wave and a fluence distribution may be decided in consideration of the positional relationship between the test object, the light irradiation region, and the acoustic wave detector, etc., and gain control may be carried out in accordance with the relation thus decided.

Next, more specific reference will be made to the construction of the biological information processing apparatus of this embodiment.

In Fig. 1, the light source 11 is a device or unit to irradiate light of a specific wave length to be absorbed by a specific component among those components which make up a living body. As the light source, there is provided at least one pulsed light source that can generate pulsed light on the order of from several to hundreds nanoseconds. A laser is desirable as the light source, but it is also possible to use a light emitting diode or the like instead of the laser. As the laser, there can be used various types of lasers such as a solid-state laser, a gas laser, a dye laser, a semiconductor laser, and the like. Here, note that although in this embodiment, an example of a single light source is shown, a plurality of light sources can be used. In the case of a plurality of light sources, in order to raise the irradiation intensity of light to be irradiated on the living body, there can be used two or more light sources which oscillate at the same wave length, or in order to measure differences in the optical property distributions according to the wave lengths, two or more light sources having different oscillation wave lengths can be used. Here, note that if a dye of which the oscillating wave length is convertible and OPO (Optical Parametric Oscillators) can be used as the light source (s), it will also become possible to measure the differences in the optical property distributions depending upon the wave lengths. It is preferable that the wave lengths to be used be in a range of from 700 nm to 1,100 nm in which there is limited absorption in the living body. However, in cases where an optical property distribution of the living body tissue relatively near a surface of the living body is obtained, it is also possible to use a wave length range, such as for example a range of from 400 nm to 1,600 nm, wider than the above-mentioned wave length range.

It is also possible to make the light 12 irradiated from the light sources propagate by using an optical waveguide or the like. Though not illustrated in Fig. 1, it is preferable to use optical fiber as the optical waveguide. In the case of using optical fiber, it is also possible to guide the light to the surface of the living body by the use of a plurality of optical fibers for the individual light sources, respectively, or light beams from the plurality of light sources may be led to a single optical fiber, and all the light beams may be guided to the living body only by using the single optical fiber. The optical device 14 is mainly composed optical components such as, for example, mirrors that reflect light, lenses that condense and expand light or change the shape of light, and the like. As such optical components, anything can be used that is able to irradiate the light 12 emitted from the light source(s) to the light irradiation region 13A on the surface of the test object in a desired shape.

The biological information processing apparatus of this embodiment is intended to make the diagnosis of malignant tumors, blood vessel diseases of humans and/or animals, the progress observation of a chemical treatment, and so on. Therefore, as the test object 13, an object to be diagnosed such as a breast, a finger, a limb (hand, foot), etc., of a human body or an animal, etc., is assumed. Also, as the light absorber, there can be applied or used those which exhibit a high absorption coefficient within the test object, and if a human body is an object to be measured, for example, such things correspond to hemoglobin, a blood vessel or a malignant tumor, which contains a lot of hemoglobin.

The acoustic wave detector (probe) 17 detects an acoustic wave (ultrasonic wave) generated from an object which has absorbed a part of energy of light propagated in the living body, and converts it into an electrical signal (detection signal). As the acoustic wave detector, there can be used any type of acoustic wave detector such as a transducerusingapiezo-electricphenomenon, a transducer using the resonance of light, a transducer using the change of capacitance, and so on, as long as an acoustic wave signal can be detected.

In this embodiment, there is shown an example of probes in which a plurality of acoustic wave detectors are arranged on the surface of the living body, but the same effect will be obtained if an acoustic wave can be detected at a plurality of places, so a single acoustic wave detector may be used to scan on a living body surface in a two-dimensional manner. Also, it is desirable to use an acoustic impedance matching agent such as gel, water or the like for suppressing the reflection of sonic waves, which is arranged between each acoustic wave detector 17 and the test object.

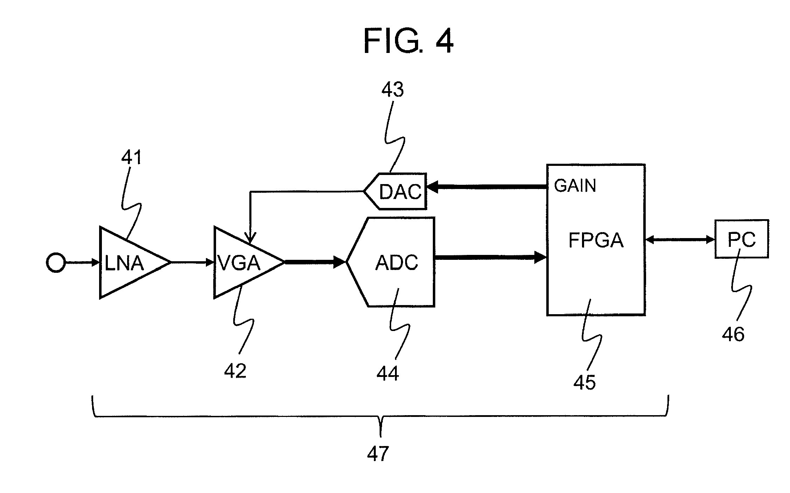

The electronic control system 18 amplifies an electrical signal obtained from each acoustic wave detector 17, and converts it from an analog signal into a digital signal. Fig. 4 shows a construction example of a circuit system 47 which the electronic control system 18 is equipped with.

First, all the photoacoustic signals detected by the acoustic wave detectors are amplified in a uniform manner by means of a low noise amplifier 41 (LNA). After that, each photoacoustic signal is amplified with a gain corresponding to the detection time thereof by means of a variable gain amplifier 42 (VGA), which corresponds to an amplifier of the present invention. Here, a personal computer (PC) 46 represents an example of a circuit system that can change the gain of VGA 42 in an arbitrary manner. The PC 46 stores a data file which defines the magnitude of a gain with respect to the detection time of each acoustic wave. For example, it is desirable that a plurality of kinds of data files have been prepared according to the kind of the test object, the positional relationship among the test object, the light irradiation region and each acoustic wave detector, etc. Then, a suitable data file corresponding to a measuring condition is transmitted from the PC 46 to a field programmable gate array 45 (FPGA). The data is sent from the FPGA 45 to a digital analog converter 43 (DAC), where it is converted from digital data into analog data. Further, the analog data is sent to a VGA 42. The VGA 42 amplifies each photoacoustic signal with a gain corresponding to the analog data thereof. That is, in this embodiment, the data files in the PC 46, the FPGA 45, and the DAC 43 together constitute a control part of the present invention. The analog signal amplified by the VGA 42 is converted into digital data by an analog digital converter 44 (ADC), and then is sent to the FPGA 45, where it is subjected to desired processing, after which it is sent to the PC 46 which is a signal processing device. If the magnitude of a gain with respect to the detection time is set so as to correct a pseudo fluence distribution in the test object, light absorbers of the same shape, size and absorption coefficient can be expressed with almost the same contrast in a biological information image finally obtained.

Here, note that the circuit arrangement shown in Fig. 4 is one example. As the electronic control system 18, there can be used any circuit that can amplify a signal detected by each acoustic wave detector 17 with a gain corresponding to a fluence distribution in the test object and can convert it into digital data.

As the signal processing device 19 of Fig. 1, anything may be used as long as it can store the digital data obtained from the electronic control system 18 and convert it into image data of an optical property distribution. Moreover, it is preferable to use one that can estimate a deliberation distribution in the test object from the shape and the light irradiation distribution of the test object, and an average optical constant of the test object. For example, there can be used a computer or the like which can analyze a variety of data. As the display device 20, anything can be used if it can display image data created by the signal processing device 19. For example, a liquid crystal display or the like can be used.

Here, note that in cases where light of a plurality of wave lengths is used, it is also possible to image the concentration distribution of substances which constitute the living body by calculating absorption coefficient distributions in the test object with respect to the individual wave lengths, respectively, and comparing the values thus obtained with wavelength dependencies inherent in those substances which constitute living body tissues. As the substances which constitute the living body tissues, there are assumed glucose, collagen, oxidized and reduced hemoglobin, etc.

According to the construction of the present invention as described above, in photoacoustic tomography, it is possible to obtain an image on which the influence of a fluence distribution in a test object is reduced. In addition, it is possible to image light absorbers of the same size, shape and absorption coefficient with almost the same contrast, without depending on the existing positions thereof. As a result, it becomes possible to image an optical property distribution (in particular, an absorption coefficient distribution) in a living body in an accurate manner.

[Second Embodiment]

In a second embodiment, reference will be made to a construction example in which an absorption coefficient distribution in the form of an optical property distribution is calculated from temporal change information of sound pressure obtained in cases where light irradiation is carried out in the same direction as the location of an acoustic wave detector.

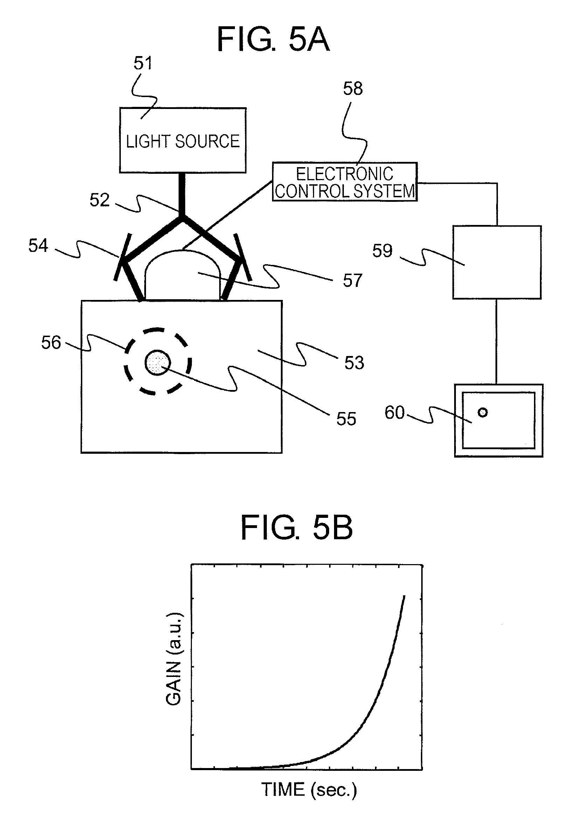

Fig. 5A illustrates a view explaining a construction example of a biological information processing apparatus in this embodiment. For the purpose of diagnosing various diseases such as malignant tumors, Alzheimer's disease, carotid artery plaque, etc., by the use of a contrast medium, the biological information processing apparatus of this embodiment serves to make it possible to image the accumulated place or location of the contrast medium introduced into a living body, and concentration distributions therein.

The biological information processing apparatus is provided with a light source 51, an optical device 54 such as mirrors, an acoustic wave detector 57, an electronic control system 58, a signal processing device 59, and a display device 59. As these components, there can be used the same as employed in the first embodiment (Fig. 1). In Fig. 5A, 52 denotes light irradiated from the light source 51, 53 denotes a test object, 55 denotes a contrast medium (light absorber) in the test object, and 56 denotes an acoustic wave generated by light irradiation. Here, note that as the contrast medium 55, there are typically used indocyanine green (ICG), goldnanoparticles, etc., are used, but any substance may be used as long as it emits an acoustic wave by being irradiated with pulsed light.

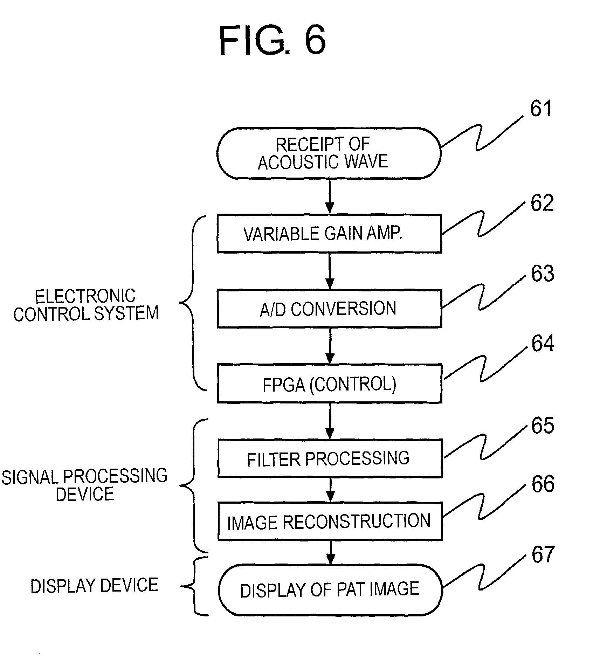

Fig. 6 illustrates the flow of signal processing for an acoustic wave signal detected by the acoustic wave detector 57.

The acoustic wave 56 generated from the contrast medium 55 is converted into an electrical signal based on the temporal change of sound pressure by means of the acoustic wave detector 57 (step 61). The signal is amplified in accordance with a pseudo fluence distribution of the test object by means of a variable gain amplifier in the electronic control system 58, as in the first embodiment (step 62). Thereafter, the signal thus amplified is subjected to analog-to-digital conversion processing by means of an ADC (step 63), and is then sent to an FPGA (step 64). The signal thus sent to the FPGA is subjected to desired processing, after which it is sent to the signal processing device (PC) 59, where it is converted into an image representing an absorption coefficient distribution of the test object by filtering processing (step 65) for noise reduction, and/or by image reconstruction processing such as phasing addition (step 66). Then, the signal thus processed is finally displayed as an image on the display device 59 (step 67).

In cases where the acoustic wave detector 57 is arranged at the same side as the light irradiation region as in this embodiment, gain control different from that in the first embodiment is required. The fluence distribution in the test object becomes a distribution that is attenuating almost exponentially as it goes away from the light irradiation region. Therefore, in this embodiment, it is necessary to provide a gain, which increases exponentially with respect to the detection time, as shown in Fig. 5B, to the acoustic wave signal detected by the acoustic wave detector 57. By performing image reconstruction with the use of the acoustic wave signal amplified in this manner, it becomes possible to obtain an image based not on an initial sound pressure distribution but on an absorption coefficient value distribution as shown in Fig. 3D. Here, note that by further correcting the fluence distribution of the test object by means of the signal processing device, it is also possible to improve the accuracy of the absorption coefficient distribution to a more extent.

[Third Embodiment]

In the first embodiment, light is irradiated to a region which is sufficiently larger than an imaging region, so it is assumed that light propagates through the interior of the test object like a plane wave. In this embodiment, however, it is presented that even in cases where such an assumption does not hold, the gain of the amplifier is controlled based on a fluence distribution in a living body.

In this embodiment, a step of calculating or determining a fluence distribution in a test object, and a step of determining a change in the gain of the above-mentioned amplifier with respect to the detection time of an acoustic wave in each element of an ultrasonic detector based on the fluence distribution are carried out.

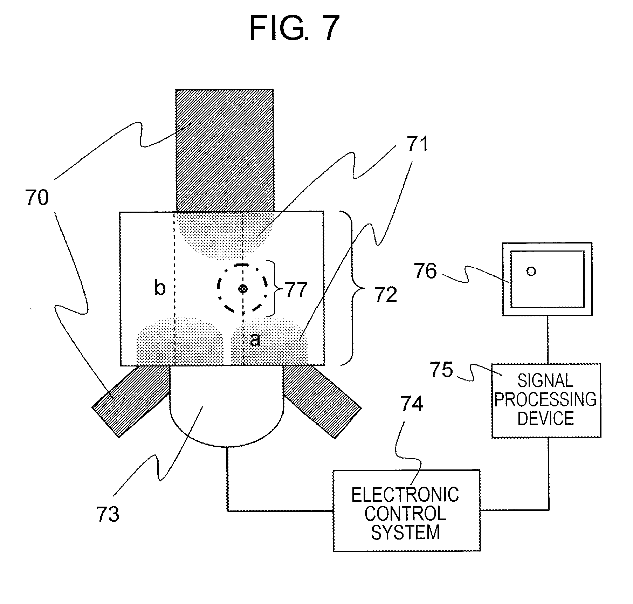

A specific embodiment will be described by using Fig. 7, Fig. 8A through Fig. 8D, and Fig. 9. Fig. 7 is a biological information processing apparatus showing an example of a third embodiment of the present invention. A light source and an optical system are the same as those in the first and second embodiments, and hence are omitted. In Fig. 7, light 70 is irradiated to a test object 71 from a side of an acoustic wave detector 73 and an opposite side thereof. 73 denotes an acoustic wave detector, 74 denotes an electronic control system, 75 is a signal processing device, and 76 is a display device.

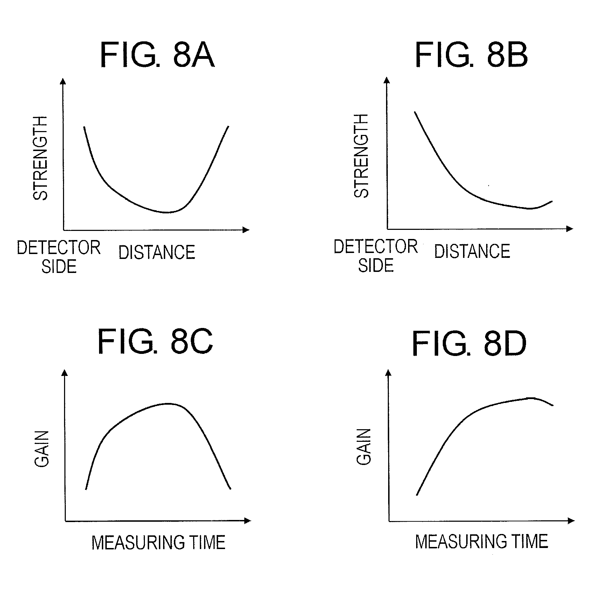

When light irradiation is carried out from a plurality of directions as in this embodiment, it becomes impossible to express a fluence distribution 71 within the test object by the use of a model which decreases exponentially in the depth direction in a simple way. For example, a fluence distribution on an axis a in Fig. 7 becomes as shown in Fig. 8A. That is, the strength of light becomes the highest in the vicinity of the acoustic wave detector 73 side of the test object, and in the vicinity of the opposite side thereof. On the other hand, a fluence distribution on an axis b in Fig. 7 becomes as shown in Fig. 8B. That is, the strength of light becomes large at the acoustic wave detector 73 side of the test object and is decreasing in accordance with the increasing distance from there. In this manner, a fluence distribution changes greatly depending on the location of its axis. Such complicated fluence distributions of the test object are estimated by means of the signal processing device 75.

The acoustic wave 77 generated from the light absorber by light irradiation is detected by the acoustic wave detector 73. The electronic control system 74 performs amplification processing adapted to the gain data calculated based on the fluence distribution estimated by the signal processing device 75 on the detection signal. The detection signal thus amplified is converted from analog data into digital data by means of the electronic control system 74, after which it is transmitted to the signal processing device 75, and is converted there into an image which represents an absorption coefficient distribution of the test object. This image is transmitted to the display device 76, and is displayed thereon.

Next, reference will be made to an estimation method of a fluence distribution. As a calculation technique for a fluence distribution, there can be used a Monte Carlo method, a finite element method, etc. In addition to such numerical calculation methods, a fluence distribution can also be calculated from an analytical solution, in cases where the living body is fixed to a certain specific shape, and in the case of a specific light irradiation condition, e.g., point irradiation or in the case of uniform broad light being irradiated into a broad range, or the like. Upon calculating a fluence distribution, an arrangement of the light irradiation region with respect to the test object, an amount of irradiation light in the light irradiation region, and optical coefficients (optical property values) such as light absorption or light scattering in the living body are required. For example, in cases where the test object is a human being, an average optical coefficient in the living body, which has been beforehand decided according to the age of the test object, the wave length of light irradiated, or the like, is used for the calculation of a fluence distribution. Here, note that the "average" optical property value in this description means an optical property value "at the time of assuming that an optical property in a living body is uniform", i.e., a background optical property value.

In addition, the signal processing device 75 may be provided, as a fluence distribution determination part of the present invention, with a table (memory) that stores a plurality of pseudo fluence distributions calculated in advance. The pseudo fluence distributions are data representing fluence distributions in the living body, and are calculated in advance about a variety of possible living body shapes and a variety of possible optical coefficients. As a calculation technique for fluence distributions, there can be used a Monte Carlo method, a finite element method, etc. In addition to such numerical calculation methods, fluence distributions can also be calculated from analytical solutions. Moreover, when an arrangement of the light irradiation region with respect to the test object, an amount of irradiation light in the light irradiation region, etc., are inputted, a fluence distribution corresponding to such a condition can be selected from a plurality of pseudo fluence distributions in the above-mentioned table.

Now, reference will be made to how to decide gains from an estimated fluence distribution. The amount of light on the axis a in Fig. 7 is estimated as shown in Fig. 8A. An X axis in Fig. 8A represents the distance, and a Y axis therein represents the strength of the amount of light. In such a case, the gain of a detection element of an acoustic wave detector lying on the axis a becomes the reciprocal of the fluence distribution thereof. That is, it becomes as shown in Fig. 8C. In Fig. 8C, an X axis represents time, and a Y axis represents gain. The conversion from distance on the X axis of Fig. 8A into time on the X axis of Fig. 8C can be made by dividing the distance by an average sound velocity in the test object. Similarly, the amount of light on the axis b of Fig. 7 is estimated as shown in Fig. 8B, so the gain of a detection element of an acoustic wave detector lying on the axis b becomes as shown in Fig. 8D. That is, in cases where a fluence distribution of the test object has a distribution in the direction of a flat surface of the test object (a direction parallel to a detection plane of the acoustic wave detector), gains given to individual detection elements of the acoustic wave detector differ for each of the detection elements. As for a gain given to each of the detection elements, it is desirable to give a gain value proportional to a reciprocal of a fluence distribution in a direction vertical to a detection plane of the detection elements.

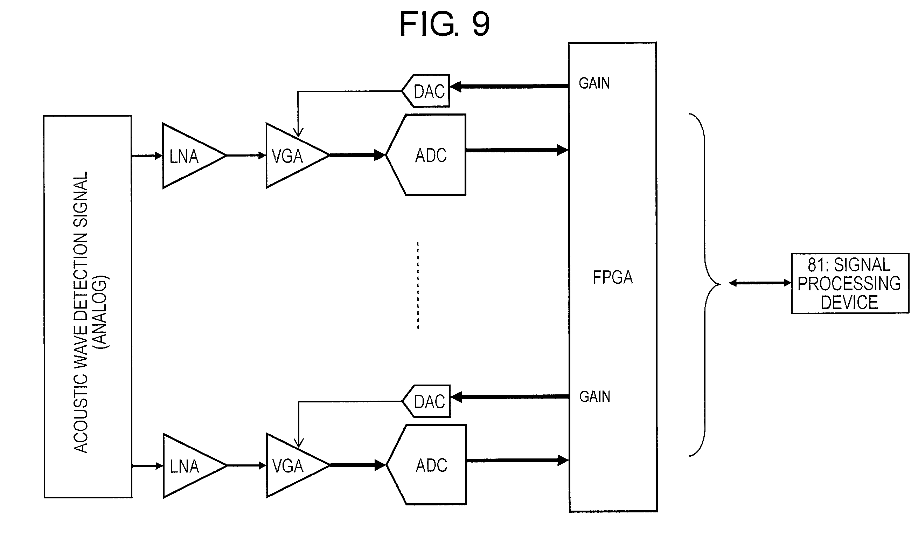

Here, note that such amplification processing of an acoustic wave detection signal can be achieved by a circuit shown in Fig. 9. In Fig. 9, 81 denotes a signal processing device which corresponds to the signal processing device 75 of Fig. 7. Although the fundamental circuit arrangement of Fig. 9 is almost the same as that of Fig. 4, it is desirable to prepare the same number of low noise amplifiers (LNA), variable gain amplifiers (VGA) and digital analog converters (DAC) as the number of detection elements present in the acoustic wave detector. Here, note that such a circuit is arranged in the electronic control system 74 in Fig. 7.

In Fig. 9, the signal processing device 81 estimates fluence distributions in the test object as shown in Fig. 8A and Fig. 8B, by means of the methods as shown above. Subsequently, the signal processing device 81 determines time dependent gain data of each of the detection elements, as shown in Fig. 8C and Fig. 8D, from the estimated fluence distributions according to the methods as shown above, and creates a data file which defines the magnitude of a gain with respect to the detection time of an acoustic wave. The data file is transmitted to the FPGA. The data of the data file is then sent from the FPGA to each DAC, where it is converted from digital data into analog data. Further, the analog data is sent to each VGA. Each VGA amplifies a photoacoustic signal received by each detection element with a gain corresponding to the analog data thereof. In this manner, an analog acoustic wave detection signal received by each of the detection elements is amplified by a gain corresponding to a light distribution of the test object. Moreover, the acoustic wave detection signal thus amplified is converted into digital data by means of each ADC, after which it is sent to the FPGA, where it is subjected to desired processing, and is then sent to the signal processing device. That is, in this embodiment, the signal processing device 81 constitutes a gain determination part and a fluence distribution determination part of the present invention, and each VGA constitutes a control part of the present invention.

By providing a different gain based on a fluence distribution to each of the detection elements in this manner, it becomes possible to obtain a reconstructed image based not on an initial sound pressure distribution but on an absorption coefficient value distribution.

**Claims**

1. A biological information processing apparatus comprising:

a light source that irradiates light to a light irradiation region on a test object;
an acoustic wave detector that detects an acoustic wave generated by a light absorber in said test object upon its absorption of the light, and outputs a detection signal;
an amplifier that amplifies the detection signal outputted from said acoustic wave detector;
a control part that controls a gain of said amplifier; and
a signal processing part that obtains information on an interior of said test object based on the signal amplified by said amplifier;
wherein said control part controls the gain of said amplifier in such a manner that a gain for a detection signal of an acoustic wave generated at a first location in said test object becomes larger as compared with a gain for a detection signal of an acoustic wave generated at a second location which exists nearer to said light irradiation region than said first location does.

2. The biological information processing apparatus according to claim 1,
wherein said control part controls the gain of said amplifier so as to correct a difference between an amount of light reaching said first location and an amount of light reaching said second location.

3. The biological information processing apparatus according to claim 1 or 2,
wherein said control part controls the gain of said amplifier with respect to said detection signal based on a fluence distribution within said test object so as to correct a difference between the amounts of light at the respective locations in said test object.

4. The biological information processing apparatus according to any one of claims 1 to 3,
wherein said acoustic wave detector is arranged in such a manner that a distance between said acoustic wave detector and said first location and a distance between said acoustic wave detector and said second location are mutually different from each other; and
said control part changes the gain of said amplifier according to a detection time of the acoustic wave by said acoustic wave detector.

5. The biological information processing apparatus according to claim 4, further comprising:

a gain determination part that determines, based on a fluence distribution within said test object, a change of the gain of said amplifier with respect to the detection time of the acoustic wave in each element of said acoustic wave detector;
wherein said control part controls the gain of said amplifier by means of an output of said gain determination part.

6. The biological information processing apparatus according to claim 5, further comprising:

a fluence distribution determination part that determines the fluence distribution within said test object based on an arrangement of the light irradiation region with respect to said test object, an amount of irradiation light in said light irradiation region, and an average optical coefficient in said test object.

7. The biological information processing apparatus according to any one of claims 1 to 4,
wherein said control part controls the gain of said amplifier in such a manner that said gain is changed in an exponential function manner with respect to the detection time of the acoustic wave by said acoustic wave detector.

8. The biological information processing apparatus according to claim 7,
   wherein an exponent in said exponential function includes an average equivalent attenuation coefficient of said test object.

9. The biological information processing apparatus according to any one of claims 1 to 8,
   wherein the information on the interior of said test object obtained by said signal processing part is an absorption coefficient distribution in the interior of said test object.

10. The biological information processing apparatus according to any one of claims 1 to 9, further comprising:

    an A/D converter that converts said signal amplified in the form of an analog signal into a digital signal.

11. A biological information processing method comprising:

    a step of detecting an acoustic wave generated by a light absorber in a test object upon its absorption of light irradiated to a light irradiation region on said test object, and outputting a detection signal;
    a step of amplifying said outputted detection signal by means of an amplifier; and
    a step of obtaining information on an interior of said test object based on the signal amplified by said amplifier;
    wherein in the step of amplifying said detection signal by means of said amplifier, the gain of said amplifier is controlled in such a manner that a gain for a detection signal of an acoustic wave generated at a first location in said test object becomes larger as compared with a gain for a detection signal of an acoustic wave generated at a second location which exists nearer to said light irradiation region than said first location does.

# FIG. 1

LIGHT SOURCE

ELECTRONIC
CONTROL SYSTEM

PC

FIG. 2A

FIG. 2B

FIG. 2C

FIG. 2D

# FIG. 3A

# FIG. 3B

# FIG. 3C

# FIG. 3D

EP 2 319 415 A1

FIG. 4

# FIG. 5A

51

LIGHT SOURCE

58

ELECTRONIC CONTROL SYSTEM

52

54

57

59

56

53

55

60

# FIG. 5B

GAIN (a.u.)

TIME (sec.)

# FIG. 6

ELECTRONIC CONTROL SYSTEM

- RECEIPT OF ACOUSTIC WAVE — 61
- VARIABLE GAIN AMP. — 62
- A/D CONVERSION — 63
- FPGA (CONTROL) — 64

SIGNAL PROCESSING DEVICE

- FILTER PROCESSING — 65
- IMAGE RECONSTRUCTION — 66

DISPLAY DEVICE

- DISPLAY OF PAT IMAGE — 67

FIG. 7

FIG. 8A

FIG. 8B

FIG. 8C

FIG. 8D

FIG. 9

81: SIGNAL
PROCESSING
DEVICE

FPGA

GAIN

GAIN

DAC

ADC

DAC

ADC

VGA

VGA

LNA

LNA

ACOUSTIC WAVE DETECTION SIGNAL
(ANALOG)

## INTERNATIONAL SEARCH REPORT

| International application No. |
| --- |
| PCT/JP2009/064857 |

**A.   CLASSIFICATION OF SUBJECT MATTER**
A61B8/00(2006.01)i, G01N29/00(2006.01)i

According to International Patent Classification (IPC) or to both national classification and IPC

**B.   FIELDS SEARCHED**

Minimum documentation searched (classification system followed by classification symbols)
A61B8/00, G01N29/00

Documentation searched other than minimum documentation to the extent that such documents are included in the fields searched
Jitsuyo Shinan Koho          1922–1996    Jitsuyo Shinan Toroku Koho    1996–2009
Kokai Jitsuyo Shinan Koho    1971–2009    Toroku Jitsuyo Shinan Koho    1994–2009

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
| --- | --- | --- |
| Y<br>A | JP 06-296612 A  (Hamamatsu Photonics Kabushiki Kaisha),<br>25 October 1994 (25.10.1994),<br>particularly, fig. 2<br>(Family: none) | 1,4,7,10,11<br>2,3,5,6,8,9 |
| Y<br>A | JP 02-289236 A  (Yokogawa Medical Systems, Ltd.),<br>29 November 1990 (29.11.1990),<br>claim 1<br>(Family: none) | 1,4,7,10,11<br>2,3,5,6,8,9 |
| A | JP 2006-521869 A  (Glucon, Inc.),<br>28 September 2006 (28.09.2006),<br>entire text; all drawings<br>& US 2008/0194929 A1    & WO 2004/086965 A1 | 1-11 |

☒   Further documents are listed in the continuation of Box C.        ☐   See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    13 October, 2009 (13.10.09) | Date of mailing of the international search report<br>    27 October, 2009 (27.10.09) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/064857 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2008-073341 A  (Toshiba Corp.),<br>03 April 2008 (03.04.2008),<br>entire text; all drawings<br>(Family: none) | 1-11 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. Xu ; L. V. Wang.** Photoacoustic imaging in biomedicine. *Review of scientific instruments,* 2006, vol. 77, 041101